(19) Europäisches Patentamt
European Patent Office
Office européen des brevets

(11) **EP 2 752 154 B1**

(12) **EUROPEAN PATENT SPECIFICATION**

(45) Date of publication and mention
of the grant of the patent:
**11.07.2018 Bulletin 2018/28**

(51) Int Cl.:
*A61B 5/02* *(2006.01)*    *A61B 5/08* *(2006.01)*

(21) Application number: **13760831.1**

(86) International application number:
**PCT/CN2013/071291**

(22) Date of filing: **01.02.2013**

(87) International publication number:
**WO 2013/135120 (19.09.2013 Gazette 2013/38)**

(54) **METHOD AND SYSTEM FOR OBTAINING PHYSIOLOGICAL SIGNAL PERIOD**

VERFAHREN UND SYSTEM FÜR DEN ERHALT VON PHYSIOLOGISCHEN SIGNALPERIODEN

PROCÉDÉ ET SYSTÈME D'OBTENTION DE PÉRIODE DE SIGNAL PHYSIOLOGIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **12.03.2012 CN 201210063536**

(43) Date of publication of application:
**09.07.2014 Bulletin 2014/28**

(73) Proprietor: **Yang, Song
Guangzhou, Guangdong (CN)**

(72) Inventors:
• **LIU, Xufang
Shenzhen
Guangdong 518000 (CN)**

• **YANG, Song
Shenzhen
Guangdong 518000 (CN)**

(74) Representative: **Manitz Finsterwald Patentanwälte
PartmbB
Martin-Greif-Strasse 1
80336 München (DE)**

(56) References cited:
**CN-A- 1 525 395          CN-A- 101 564 300
CN-A- 101 732 050      CN-A- 102 613 964
FR-A5- 2 064 874         JP-A- 2007 181 628
US-A- 5 076 281          US-A- 5 321 350
US-A1- 2004 186 388   US-A1- 2009 204 011**

EP 2 752 154 B1

**EP 2 752 154 B1**

**Description**

**BACKGROUND**

1. Technical Field

The present disclosure generally relates to technologies for obtaining physiological signals, and more particularly, to a method and a system for obtaining a cycle of a physiological signal.

2. Description of Related Art

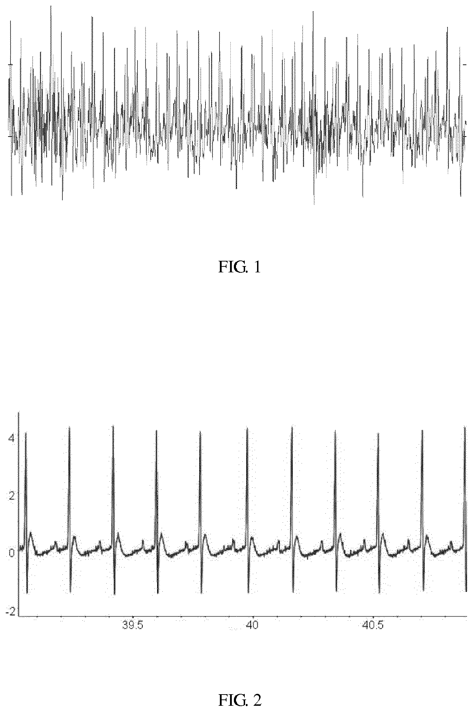

Important physiological signals of a human body, such as heartbeat signals and respiration signals, were always obtained by collecting and processing electromyography signals. A signal collecting device needed to contact in tight with human skin for acquiring clear signals which are thereafter amplified and processed. A cycle of the physiological signal obtained in the above way can be obtained as follows: a simple threshold at first is used to set a shaping and obtain a flag bit related to the cycle, then the cycle is obtained by calculating the flag bit of the cycle.
A micro-motion signal of a human body such as a heartbeat signal or a respiration signal (as shown in FIG. 1) sensed by a micro-motion sensor, has a waveform which is totally different from that of a bioelectric signal (as shown in FIG. 2). The waveform includes a different number of wave beams in a skip cycle, a magnitude of the waveform changes linearly according to the cycle, and the cycle cannot be calculated by threshold shaping or simple Fourier transformation. At present, the most common method to calculate the cycle is the real-time heartbeat rate recognition. However, the real-time heartbeat rate recognition method uses the auto-correlation function with a large amount of computation, making it hard to operate the real-time heartbeat rate recognition method in cheap ARMs and thus increasing the cost of obtaining the cycle.
US2004/186388A1 discloses a method for detecting R-waves in an electrical signal generated by the heart. The method processes digitized samples of an electrocardiogram signal to determine the timing of the top of the R-wave that coincides with the maximum depolarization activity in the ventricles of the heart. The detection method repetitively determines the slope of the EKG signal within a predetermined time period equal to the normal R-wave rise time (approximately 30 ms). Samples of the electrocardiogram signal are received one-by-one and stored in a multi-sample buffer (e.g., 8 samples at a sampling rate of 250 samples per second) in order to calculate the instantaneous slope of the signal. If the amplitude of a detected peak is greater than the current threshold value, it is considered to be an R-wave. The threshold value against which the newly detected peak is compared is a fraction (e.g. two-thirds) of the previously detected peak amplitude.
US2009/204011A1 discloses a pulse wave period measuring apparatus including a pulse wave detecting unit configured to detect a pulse wave of a subject, a value detecting unit configured to detect a maximum value and a minimum value in a predetermined period of the pulse wave, a reference value calculating unit configured to calculate one or more reference values between the maximum value and the minimum value in the predetermined period, based on one or more internally dividing ratios common to plural predetermined periods of the pulse wave.
JP2007181628A discloses a pulsation interval calculator and a calculation method. In the calculator/ method, a pulsation time interval between two specific time points tR when the value P of pulse waves becomes a specified value PR is calculated; the time point t when the value P of pulse wave has reached a level where the minimum value PL has a certain ratio PR to the maximum value PH is selected as the specific time point tR.

**SUMMARY**

The main object of the present disclosure is to provide a method for obtaining a cycle of a physiological signal which improves the efficiency of obtaining the cycle of the physiological signal and reduces the cost of obtaining the cycle.
The method for obtaining a cycle of a physiological signal provided in the present disclosure includes:

receiving a physiological signal value and a register value, comparing the physiological signal value with the register value, and reserving one of the physiological signal value and the register value; determining the physiological signal value with a time duration thereof reaching a given set time to be an extreme value; and restarting the procedure and determining a next extreme value; and
obtaining a cycle of the physiological signal by calculating a time difference between the extreme value and the next extreme value.

Preferably, the method further includes the following steps:

judging whether two cycles respectively obtained in a maximum value way and in a minimum value way in a time duration are close to each other; and

determining an average of the two cycles to be the cycle of the physiological signal if the two cycles are close to each other.

Preferably, the step of receiving a physiological signal value and register value and comparing the physiological signal value with the register value; determining the physiological signal value with a time duration thereof reaching a given set time to be an extreme value; and restarting the procedure and determining a next extreme value includes:

receiving the physiological signal value, starting a counter a and adding an unit value to the counter a, comparing the physiological signal value with the register value; if the physiological signal value is greater than/less than the register value, replacing the register value with the physiological signal value, adding a count value of the counter a to an accumulator b, and clearing the counter a to zero; if the physiological signal value is less than/greater than the register value, keeping receiving a next heartbeat signal voltage; determining the corresponding physiological signal value to be an extreme value when the count value of the counter a reaches a value corresponding to the give set time, outputting a value accumulated in the accumulator b, clearing the counter, a register, and the accumulator b, and keeping determining the next extreme value; the extreme value and the next extreme value being respectively a maximum value or a minimum value.

Preferably, the step of obtaining the cycle of the physiological signal by calculating a time difference between the extreme value and the next extreme value includes:

obtaining the cycle of the physiological signal by adding a time required for reaching the value accumulated in the accumulator b and the given set time.

Preferably, the given set time is longer than a half cycle of an upper limit of a recognition range of the cycle of the physiological signal.

The present disclosure further provides a system for obtaining a cycle of a physiological signal, including:

an extreme value judging unit, configured for receiving a physiological signal value and a register value, comparing the physiological signal value with the register value, and reserving one of the physiological signal value and the register value; determining the physiological signal value with a time duration thereof reaching a given set time to be an extreme value; and restarting the procedure and determining a next extreme value;

a cycle calculating unit, configured for obtaining a cycle of the physiological signal by calculating a time difference between the extreme value and the next extreme value.

Preferably, the system further includes:

a close cycle judging unit, configured for judging whether two cycles respectively obtained in a maximum value way and in a minimum value way in a time duration are close to each other; and

a cycle determining unit, configured for determining an average of the two cycles to be the cycle of the physiological signal if the two cycles are close to each other.

Preferably, the extreme value judging unit is configured for: receiving the physiological signal value, starting a counter a and adding an unit value to the counter a, comparing the physiological signal value with the register value; if the physiological signal value is greater than/less than the register value, replacing the register value with the physiological signal value, adding a count value of the counter a to an accumulator b, and clearing the counter a to zero; if the physiological signal value is less than/greater than the register value, keeping receiving a next heartbeat signal voltage; determining the corresponding physiological signal value to be an extreme value when the count value of the counter a reaches a value corresponding to the give set time, outputting a value accumulated in the accumulator b, clearing the counter a, a register, and the accumulator b, and keeping determining the next extreme value; the extreme value and the next extreme value being respectively a maximum value or a minimum value.

Preferably, the cycle calculating unit is configured for: obtaining the cycle of the physiological signal by adding a time required for reaching the value accumulated in the accumulator and the given set time.

Preferably, the given set time is longer than a half cycle of an upper limit of a recognition range of the cycle of the physiological signal.

In the present disclosure, the cycle of the physiological signal can be obtained by the extreme value recognition algorithm, which is simple, fast, of high efficiency, and of high reliability; furthermore, the requirements about the amplification of the physiological signal, the filtering of the physiological signal, and the analogy-to-digital conversion are relatively low and the data process is relatively simple, thus, the hardware cost can be greatly reduced.

## DESCRIPTION OF THE DRAWINGS

Many aspects of the embodiments can be better understood with reference to the following drawings. The components in the drawings are not necessarily dawns to scale, the emphasis instead being placed upon clearly illustrating the principles of the embodiments. Moreover, in the drawings, like reference numerals designate corresponding parts throughout the several views.

Fig. 1 is a schematic view of a waveform of a signal obtained by a piezoelectric sensor in a related art;
FIG. 2 is a schematic view of a waveform of a bioelectric signal in the related art;
FIG. 3 is a flow chart of a method for obtaining a cycle of a physiological signal in accordance with an embodiment of the present disclosure;
FIG. 4 is a flow chart of a method for obtaining a cycle of a physiological signal in accordance with another embodiment of the present disclosure;
FIG. 5 is a schematic view of a system for obtaining a cycle of a physiological signal in accordance with an embodiment of the present disclosure; and
FIG. 6 is a schematic view of a system for obtaining a cycle of a physiological signal in accordance with another embodiment of the present disclosure.

## DETAILED DESCRIPTION

The disclosure is illustrated by way of example and not by way of limitation in the figures of the accompanying drawings in which like references indicate similar elements. It should be noted that references to "an" or "one" embodiment is this disclosure are not necessarily to the same embodiment, and such references mean at least one.

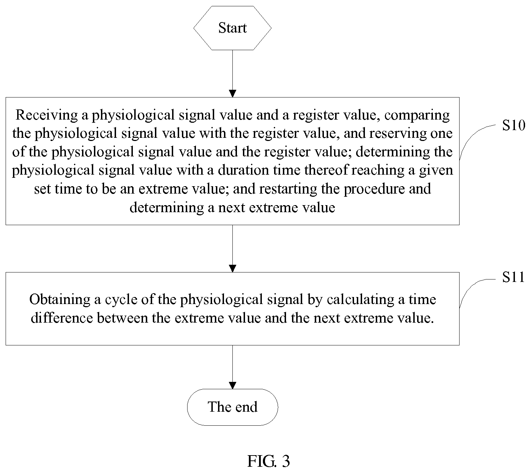

Referring to FIG. 3, a method for obtaining a cycle of a physiological signal in accordance with an embodiment of the present disclosure is provided. The method includes:

step S10, receiving a physiological signal value and a register value, comparing the physiological signal value with the register value, and reserving one of the physiological signal value and the register value; determining the physiological signal value with a duration time thereof reaching a given set time to be an extreme value; and restarting the procedure and determining a next extreme value; and
step S11, obtaining a cycle of the physiological signal by calculating a time difference between the extreme value and the next extreme value.

In the embodiment, the physiological signal can be a respiration signal or a heartbeat signal, the physiological signal value can be a specific value such as a voltage, and the physiological signal can be obtained by a piezoelectric sensor. In the above method for obtaining the cycle of the physiological signal, a micro-motion sensor such as the piezoelectric sensor can be used for obtaining a micro-motion signal of a human body when the human body is in a quiet state, and the cycle of the physiological signal such as the respiration signal and/ or the heartbeat signal can be directly determined in a carrier signal.

The step S10 specifically includes: receiving one physiological signal value, starting a counter a and adding an unit value to the counter a, and comparing the physiological signal value with the register value; if the physiological signal value is greater than/ less than the register value, replacing the register value with the physiological signal value, adding a count value of the counter a to an accumulator b, and clearing the counter a to zero; if the physiological signal value is less than/ greater than the register value, keeping receiving a next voltage of the physiological signal; when the count value of the counter a reaches a value corresponding to the given set time, determining the physiological signal value to be an extreme value, outputting the value accumulated in the accumulator b, and clearing the counter a, a register, and the accumulator b, and continuously determining a next extreme value. The register value can be equal to zero or the reserved physiological signal value.

The step S11 can specifically include: obtaining the cycle of the physiological signal by adding the time required for reaching the value accumulated in the accumulator b and the given set time.

The cycle of the physiological signal can be obtained by an extreme value recognition algorithm. The extreme value can be a maximum value or a minimum value, that is, the current extreme value and the next extreme value can respectively be a maximum value or a minimum value. The maximum value recognition algorithm can be: timing a duration when one received physiological signal value is kept greatest, and determining the received physiological signal value to be a maximum value in one cycle when the timer reaches the given set time. The time difference (time length) between the maximum value (the extreme value) and a next maximum value (the next extreme value) can be calculated. The minimum value recognition algorithm can be: timing a duration when one received physiological signal is kept smallest, and determining the received physiological signal value to be a minimum value in one cycle when the timer reaches the given set time. Furthermore, the time difference between the minimum value (the extreme value) and a next minimum value (the next extreme value) can be calculated. The cycle of the physiological signal obtained by the maximum value recognition algorithm and the cycle of the physiological signal obtained by the minimum value recognition algorithm are respectively obtained according to the given set time and the time difference between the two maximum values or the time difference between the two minimum values. The given set time can be set according to the specific physiological signal, for example, in the embodiment the given set time can be longer than a half of the cycle of the physiological signal.

The method for obtaining the above physiological signal can further be described as follows based on the following example in which the cycle of the physiological signal is obtained in the maximum value way.

At first, receiving a voltage of a heartbeat signal outputted from the piezoelectric sensor, starting the counter a and adding 1 to the counter a, comparing the count value of the counter a with the register value in the register; if the count value is greater than the register value, replacing the register value with the count value, adding the count value of the counter a to the accumulator b, and clearing the counter a to zero; if the count value is less than the register value; keeping receiving the next voltage of the heartbeat signal.

Outputting the value accumulated in the accumulator b, clearing the counter a, the register, and the accumulator b, and starting a new cycle when the count value of the counter a reaches a corresponding value. Each heartbeat signal received in the new cycle is compared with the register value 0 in the register. The formula for calculating the cycle of the above heartbeat signal is expressed as:

$$\text{Cycle (s)} = (\text{the value accumulated in the accumulator b}/ \text{ a receiving speed of the physiological signal}) + \text{the given set time.}$$

Since the constant of the time duration (that is, the given set time) when the physiological signal is kept being greatest needs to be previously set, thus, an upper limit of the recognition cycle can be the reciprocal of the given set time.

For example, if the frequency of the heartbeat signal ranges from 0.7 Hz to 1.6 Hz, then the given set time of the maximum value is 0.55 seconds (the given set time of the minimum value is 0.6 seconds). If a received heartbeat signal is kept being greatest in the register for 0.55 seconds, then the counter a starts to count. Adding 1 to the counter a when a new heartbeat signal is inputted into the comparator. If the voltage of the new heartbeat signal is greater than the previous register value, storing the voltage of the new heartbeat signal in the register and replacing the previous register value with the voltage, adding the count value of the counter a to the accumulator b, and clearing the counter to zero. If the voltage of the new heartbeat signal is less than the previous register value stored in the register, adding 1 to the counter, continuing the cycle of comparing the voltage of the heartbeat signal, outputting a value B accumulated in the accumulator b until the count value of the counter a is equal to the number of the voltages (for example, 300) of the heartbeat signals received in 0.55 seconds, and adding 0.55 seconds and the time required for reaching the value B accumulated in the accumulator b to acquire the cycle of the heartbeat signal.

Supposed that the number of the received heartbeat signals counted by the counter a in 0.55 seconds when one heartbeat signal is kept being greatest is 275, and the speed of receiving the heartbeat signal is 500 per second, then the cycle of the heartbeat signal = (B/500) + 0.55.

According to the above method, the cycle of the physiological signal is respectively obtained in the maximum value way and in the minimum way in a time duration, thereafter the more accurate cycle of the physiological signal can be obtained according to the cycle of the physiological signal obtained in the maximum value way and the cycle of the physiological signal obtained in the minimum value way.

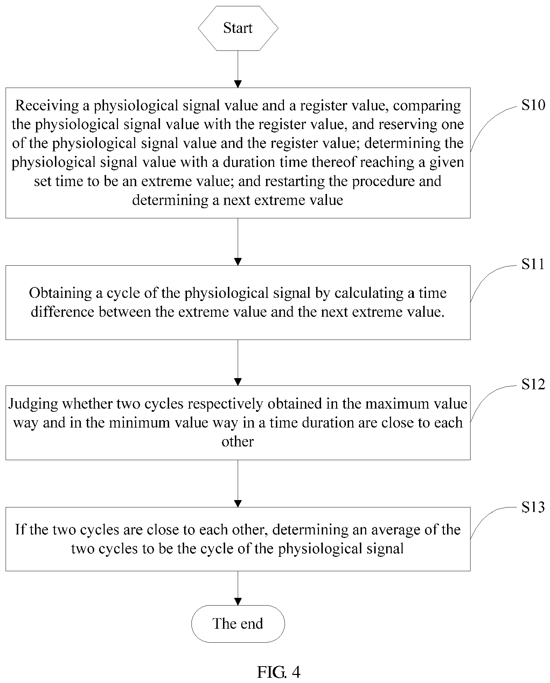

Referring to FIG. 4, in another embodiment of the present disclosure, the method for obtaining the cycle of the physiological signal described above can further include the following steps:

step S12, judging whether two cycles respectively obtained in the maximum value way and in the minimum value way in a time duration are close to each other; and

step S13, if the two cycles are close to each other, determining an average of the two cycles to be the cycle of the physiological signal.

In a given set time duration, for example, at least one cycle of the physiological signal obtained in the maximum value way and at least one cycle of the physiological signal obtained in the minimum value way are obtained. The cycles obtained in the two ways are compared with each other to judge whether the two cycles are close to each other. If the two cycles are close to each other, averaging the two cycles and determining the average of the two cycles to be the more accurate cycle of the physiological signal, otherwise ending the procedure. Whether the two cycles are close to each other or not can be judged according to the specific type of the physiological signal, for example, the time difference between two close cycles of a respiration signal is approximately 0.004 seconds and the time difference between two close cycles of a heartbeat signal is approximately 0.017 seconds.

In the above method for obtaining the cycle of the physiological signal, the cycle of the physiological signal can be obtained by the extreme value recognition algorithm, which is simple, fast, of high efficiency, and of high reliability; furthermore, the requirements about the amplification of the physiological signal, the filtering of the physiological signal, and the analogy-to-digital conversion are relatively low and the data process is relatively simple, thus, the hardware cost can be greatly reduced.

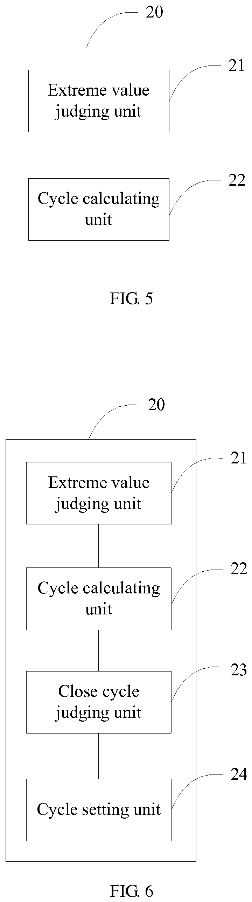

Referring to FIG. 5, a system 20 for obtaining a cycle of a physiological signal in accordance with an embodiment of the present disclosure is provided. The system 20 includes an extreme value judging unit 21 and a cycle calculating unit 22. The extreme value judging unit 21 is configured for receiving a physiological signal value and a register value, comparing the physiological signal value with the register value, and reserving one of the physiological signal value and the register value; determining the physiological signal value with a time duration thereof reaching a given set time to be an extreme value; and restarting the procedure and determining a next extreme value. The cycle calculating unit 22 is configured for obtaining a cycle of the physiological signal by calculating a time difference between the extreme value and the next extreme value.

In the embodiment, the physiological signal can be a respiration signal or a heartbeat signal, the physiological signal value can be a specific value such as a voltage, and the physiological signal can be obtained by a piezoelectric sensor. In the above system for obtaining the cycle of the physiological signal, a micro-motion sensor such as the piezoelectric sensor can be used for obtaining a micro-motion signal of a human body when the human body is in a quiet state, and the cycle of the physiological signal such as the respiration signal and/ or the heartbeat signal can be directly determined in a carrier signal.

The extreme value judging unit 21 is specifically used for: receiving one physiological signal value, starting a counter a and adding an unit value to the counter, and comparing the physiological signal value with the register value; if the physiological signal value is greater than/ less than the register value, replacing the register value with the physiological signal value, adding a count value of the counter to an accumulator b, and clearing the counter to zero; if the physiological signal value is less than/ greater than the register value, keeping receiving a next voltage of the heartbeat signal; when the count value of the counter reaches a value corresponding to the given set time, determining the physiological signal value to be an extreme value, outputting a value accumulated in the accumulator b, and clearing the counter a, a register, and the accumulator b, and continuously determining the next extreme value. The register value can be equal to zero or the reserved physiological signal value.

The cycle calculating unit 22 is specifically used for: obtaining the cycle of the physiological signal by adding a time required for reaching the value accumulated in the accumulator b and the given set time.

The cycle of the physiological signal can be obtained by an extreme value recognition algorithm. The extreme value can be a maximum value or a minimum value, that is, the current extreme value and the next extreme value can respectively be a maximum value or a minimum value. The maximum value recognition algorithm can be: timing a duration when one received physiological signal value is kept greatest, and determining the received physiological signal value to be a maximum value in one cycle when the timer reaches the given set time. The time difference (time length) between the maximum value (the extreme value) and a next maximum value (the next extreme value) can be calculated. The minimum value recognition algorithm can be: timing a duration when one received physiological signal is kept smallest, and determining the received physiological signal value to be a minimum value in one cycle when the timer reaches the given set time. Furthermore, the time difference between the minimum value (the extreme value) and a next minimum value (the next extreme value) can be calculated. The cycle of the physiological signal obtained by the maximum value recognition algorithm and the cycle of the physiological signal obtained by the minimum value recognition algorithm are respectively obtained according to the given set time and the time difference between two maximum values or the time difference between two minimum values. The given set time can be set according to the specific physiological signal, for example, in the embodiment the given set time can be longer than a half of the cycle of the physiological signal.

The system 20 for obtaining the above physiological signal can further be described as follows based on the following example in which the cycle of the physiological signal is obtained in the maximum value way.

At first, receiving a voltage of a heartbeat signal outputted from the piezoelectric sensor, starting the counter a and adding 1 to the counter a, comparing the count value of the counter a with the register value in the register; if the count value is greater than the register value, replacing the register value with the count value, adding the count value of the counter a to the accumulator b, and clearing the counter a to zero; if the count value is less than the register value; keeping receiving the next voltage of the heartbeat signal.

Outputting the value accumulated in the accumulator b, clearing the counter a, the register, and the accumulator b, and starting a new cycle when the count value of the counter a reaches a corresponding value. Each heartbeat signal received in the new cycle is compared with the register value 0 in the register. The formula for calculating the cycle of the above heartbeat signal is expressed as:

$$\text{Cycle (s)} = \text{(the value accumulated in the accumulator b/ a receiving speed of the physiological signal)} + \text{the given set time.}$$

Since the constant of the time duration (that is, the given set time) when the physiological signal is kept being greatest needs to be previously set, thus, an upper limit of the recognition cycle can be the reciprocal of the given set time.

For example, if the frequency of the heartbeat signal ranges from 0.7 Hz to 1.6 Hz, then the given set time of the maximum value is 0.55 seconds (the given set time of the minimum value is 0.6 seconds). If a received heartbeat signal is kept being greatest in the register for 0.55 seconds, then the counter a starts to count. Adding 1 to the counter a when a new heartbeat signal is inputted into the comparator. If the voltage of the new heartbeat signal is greater than the previous register value, storing the voltage of the new heartbeat signal in the register and replacing the previous register value with the voltage, adding the count value of the counter a to the accumulator b, and clearing the counter to zero. If the voltage of the new heartbeat signal is less than the previous register value stored in the register, adding 1 to the counter, continuing the cycle of comparing the voltage of the heartbeat signal, outputting a value B accumulated in the accumulator b until the count value of the counter a is equal to the number of the voltages (for example, 300) of the heartbeat signals received in 0.55 seconds, and adding 0.55 seconds and the time required for reaching the value B in the accumulator b to acquire the cycle of the heartbeat signal.

Supposed that the number of the received heartbeat signals counted by the counter a in 0.55 seconds when one received heartbeat signal is kept being greatest is 275, and the speed of receiving the heartbeat signal is 500 per second, then the cycle of the heartbeat signal = (B/500) + 0.55.

According to the above method, the cycle of the physiological signal is respectively obtained in the maximum value way and in the minimum value way in a time duration, thereafter the more accurate cycle of the physiological signal can be obtained according to the cycle of the physiological signal obtained in the maximum value way and the cycle of the physiological signal obtained in the minimum value way.

Referring to FIG. 6, in another embodiment of the present disclosure, the system can further include a close cycle judging unit 23 and a cycle setting unit 24. The close cycle judging unit 23 is used for judging whether two cycles respectively obtained in the maximum value way and in the minimum value way in a time duration are close to each other. The cycle setting unit 24 is used for setting an average of the two cycles to be the cycle of the physiological signal if the two cycles are close to each other.

In a given set time duration, at least one cycle of the physiological signal obtained in the maximum value way and at least one cycle of the physiological signal obtained in the minimum value way are obtained. The two cycles obtained in the two ways are compared with each other to judge whether the two cycles are close to each other. If the two cycles are close to each other, averaging the two cycles and determining the average of the two cycles to be the more accurate cycle of the physiological signal, otherwise ending the procedure. Whether the two cycles are close to each other or not can be judged according to the specific type of the physiological signal, for example, the time difference between two close cycles of a respiration signal is approximately 0.004 seconds and the time difference between two close cycles of a heartbeat signal is approximately 0.017 seconds.

In the above system 20 for obtaining the cycle of the physiological signal, the cycle of the physiological signal can be obtained by the extreme value recognition algorithm, which is simple, fast, of high efficiency, and of high reliability; furthermore, the requirements about the amplification of the physiological signal, the filtering of the physiological signal, and the analogy-to-digital conversion are relatively low and the data process is relatively simple, thus, the hardware cost can be greatly reduced.

Even though information and the advantages of the present embodiments have been set forth in the foregoing description, together with details of the mechanisms and functions of the present embodiments, the disclosure is illustrative only; and that changes may be made in detail, especially in matters of shape, size, and arrangement of parts within the principles of the present embodiments to the full extend indicated by the broad general meaning of the terms in which the appended claims are expressed.

**Claims**

1. A computer implemented method for obtaining a cycle of a physiological signal, the physiological signal being a respiration signal or a heartbeat signal, the method comprising:

   a maximum value recognition procedure and a minimum value recognition procedure,
   **characterized in that**
   the maximum value recognition procedure comprises:

receiving a current physiological signal value;

starting a counter a and adding a unit value to the counter a;

comparing the current physiological signal value with a register value; if the current physiological signal value is greater than the register value,

replacing the register value with the physiological signal value, adding a count value of the counter a to an accumulator b, and clearing the counter a to zero, and receiving a next physiological signal value;

if the current physiological signal value is less than the register value, receiving a next physiological signal value;

determining the corresponding physiological signal value to be an maximum value when the count value of the counter a reaches a value corresponding to a first pre-set time;

outputting a value accumulated in the accumulator b, clearing the counter a, the register, and the accumulator b;

restarting the maximum value recognition procedure and determining a next maximum value; and

obtaining a first cycle of the physiological signal by adding a time required for reaching the value accumulated in the accumulator b and the first pre-set time,

the minimum value recognition procedure comprises:

receiving a current physiological signal value;

starting a counter a and adding a unit value to the counter a;

comparing the current physiological signal value with a register value;

if the current physiological signal value is less than the register value, replacing the register value with the physiological signal value, adding a count value of the counter a to an accumulator b, and clearing the counter a to zero, and receiving a next physiological signal value;

if the current physiological signal value is greater than the register value, receiving a next physiological signal value;

determining the corresponding physiological signal value to be an minimum value when the count value of the counter a reaches a value corresponding to a second pre-set time;

outputting a value accumulated in the accumulator b, clearing the counter a, the register, and the accumulator b;

restarting the minimum value recognition procedure and determining a next minimum value; and

obtaining a second cycle of the physiological signal by adding a time required for reaching the value accumulated in the accumulator b and the second pre-set time;

the method further comprising the following steps:

judging whether the first cycle and the second cycle respectively obtained in a time duration are close to each other; and

if the first cycle and the second cycle are close to each other, determining an average of the two cycles to be the cycle of the physiological signal,

wherein the first cycle and the second cycle being close to each other means that the first cycle and the second cycle has a time difference of 0.004 seconds if the physiological signal is a respiration signal or has time difference of 0.017 seconds if the physiological signal is a heartbeat signal.

2. The method of any one from claim 1 to claim 1, wherein the first pre-set time and the second pre-set time are longer than a half cycle of an upper limit of a recognition range of the cycle of the physiological signal.

3. A system for obtaining a cycle of a physiological signal by executing the method according to claim 1 or 2, comprising:

an extreme value judging unit, configured for performing the maximum value recognition procedure and the minimum value recognition procedure;

a cycle calculating unit, configured for obtaining the first cycle of the physiological signal and the second cycle of the physiological signal,

a close cycle judging unit, configured for judging whether the first cycle and the second cycle respectively obtained in a time duration are close to each other; and

a cycle determining unit, configured for determining an average of the two cycles to be the cycle of the physiological signal if the first cycle and the second cycle are close to each other.

**Patentansprüche**

1. Computerimplementiertes Verfahren zum Erhalten eines Zyklus eines physiologischen Signals, wobei das physiologische Signal ein Beatmungssignal oder ein Herzschlagsignal ist, wobei das Verfahren umfasst:

   ein Maximalwerterkennungsverfahren und ein Minimalwerterkennungsverfahren,
   **dadurch gekennzeichnet, dass**
   das Maximalwerterkennungsverfahren umfasst:

   Empfangen eines gegenwärtigen Wertes eines physiologischen Signals;
   Starten eines Zählers a und Addieren eines Einheitswerts zu dem Zähler a;
   Vergleichen des gegenwärtigen Wertes des physiologischen Signals mit einem Registerwert;

   falls der gegenwärtige Wert des physiologischen Signals größer als der Registerwert ist, Ersetzen des Registerwerts mit dem Wert des physiologischen Signals, Addieren eines Zählwertes des Zählers a zu einem Akkumulator b und Löschen des Zählers a auf Null und Empfangen eines nächsten Wertes eines physiologischen Signals;
   falls der gegenwärtige Wert des physiologischen Signals kleiner als der Registerwert ist, Empfangen eines nächsten Wertes des physiologischen Signals;
   Bestimmen des entsprechenden Wertes des physiologischen Signals als einen Maximalwert, wenn der Zählwert des Zählers einen Wert erreicht, der einer ersten voreingestellten Zeit entspricht;
   Ausgeben eines Wertes, der in dem Akkumulator b angesammelt ist, Löschen des Zählers a, des Registers und des Akkumulators b;
   Neustarten des Maximalwerterkennungsverfahrens und Bestimmen eines nächsten Maximalwertes; und
   Erhalten eines ersten Zyklus des physiologischen Signals durch Addieren einer Zeit, die zum Erreichen des Wertes, der in dem Akkumulator b angesammelt ist, erforderlich ist, und der ersten voreingestellten Zeit,

   wobei das Minimalwerterkennungsverfahren umfasst:

   Empfangen eines gegenwärtigen Wertes des physiologischen Signals;
   Starten eines Zählers a und Addieren eines Einheitswerts zu dem Zähler a;
   Vergleichen des gegenwärtigen Wertes des physiologischen Signals mit einem Registerwert;
   falls der gegenwärtige Wert des physiologischen Signals kleiner als der Registerwert ist, Ersetzen des Registerwerts mit dem Wert des physiologischen Signals, Addieren eines Zählwerts des Zählers a zu einem Akkumulator b und Löschen des Zählers a auf Null und Empfangen eines nächsten Wertes eines physiologischen Signals;
   falls der gegenwärtige Wert des physiologischen Signals größer als der Registerwert ist, Empfangen eines nächsten Wertes des physiologischen Signals;
   Bestimmen des entsprechenden Wertes des physiologischen Signals als einen Minimalwert, wenn der Zählwert des Zählers a einen Wert erreicht, der einer zweiten voreingestellten Zeit entspricht;
   Ausgeben eines Wertes, der in dem Akkumulator b angesammelt ist, Löschen des Zählers a, des Registers und des Akkumulators b;
   Neustarten des Minimalwerterkennungsverfahrens und Bestimmen des nächsten Minimalwertes; und
   Erhalten eines zweiten Zyklus des physiologischen Signals durch Addieren einer Zeit, die zum Erreichen des Wertes, der in dem Akkumulator b angesammelt ist, erforderlich ist, und der zweiten voreingestellten Zeit;

   wobei das Verfahren ferner die Schritte umfasst:

   Beurteilen, ob der erste Zyklus bzw. der zweite Zyklus, die innerhalb einer Zeitdauer erhalten werden, eng beieinander liegen; und
   falls der erste Zyklus und der zweite Zyklus eng beieinander liegen, Bestimmen eines Durchschnitts der beiden Zyklen als den Zyklus des physiologischen Signals,

wobei das enge Beieinanderliegen des ersten Zyklus und des zweiten Zyklus bedeutet, dass der erste Zyklus und der zweite Zyklus eine Zeitdifferenz von 0,004 Sekunden aufweisen, falls das physiologische Signal ein Beatmungssignal ist, oder eine Zeitdifferenz von 0,017 Sekunden aufweisen, falls das physiologische Signal ein Herzschlagsignal ist.

2. Verfahren nach Anspruch 1, wobei die erste voreingestellte Zeit und die zweite voreingestellte Zeit länger als ein halber Zyklus einer oberen Grenze eines Erkennungsbereiches des Zyklus des physiologischen Signals sind.

3. System zum Erhalten eines Zyklus eines physiologischen Signals durch Ausführen des Verfahrens nach einem der Ansprüche 1 oder 2, umfassend:

eine Extremwertbeurteilungseinheit, die zum Ausführen des Maximalwerterkennungsverfahrens und des Minimalwerterkennungsverfahrens konfiguriert ist;
eine Zyklusberechnungseinheit, die zum Erhalten des ersten Zyklus des physiologischen Signals und des zweiten Zyklus des physiologischen Signals konfiguriert ist,
eine Einheit zum Beurteilen eines engen Zyklus, die zum Beurteilen konfiguriert ist, ob der erste Zyklus bzw. der zweite Zyklus, die innerhalb einer Zeitdauer erhalten werden, eng beieinanderliegen; und
eine Zyklusbestimmungseinheit, die zum Bestimmen eines Durchschnitts der beiden Zyklen als den Zyklus des physiologischen Signals konfiguriert ist, wenn der erste Zyklus und der zweite Zyklus eng beieinander liegen.

**Revendications**

1. Procédé mis en oeuvre en ordinateur pour obtenir un cycle d'un signal physiologique, le signal physiologique étant un signal de respiration ou un signal de battement cardiaque, le procédé comprenant :

une procédure de reconnaissance de valeur maximum et une procédure de reconnaissance de valeur minimum,
**caractérisé en ce que**
la procédure de reconnaissance de valeur maximum comprend les étapes consistant à :

recevoir une valeur actuelle d'un signal physiologique ;
démarrer un compteur a et ajouter une valeur unitaire au compteur a ;
comparer la valeur actuelle du signal physiologique avec une valeur mémorisée ;
si la valeur actuelle du signal physiologique est supérieure à la valeur inscrite, remplacer la valeur mémorisée par la valeur du signal physiologique, ajouter une valeur de décompte du compteur a à un accumulateur b, effacer le compteur a à zéro, et recevoir une valeur suivante du signal physiologique ;
si la valeur actuelle du signal physiologique est inférieure à la valeur inscrite, recevoir une valeur suivante du signal physiologique ;
déterminer que la valeur correspondante du signal physiologique est une valeur maximum quand la valeur de décompte du compteur a atteint une valeur correspondant à un premier temps préétabli ;
délivrer une valeur accumulée dans l'accumulateur b, effacer le compteur a, la mémoire, et l'accumulateur b ;
redémarrer la procédure de reconnaissance de valeur maximum et déterminer une valeur maximum suivante ; et
obtenir un premier cycle du signal physiologique en ajoutant un temps requis pour atteint la valeur accumulée dans l'accumulateur b et le premier temps préétabli,

la procédure de reconnaissance de valeur minimum comprend les étapes consistant à :

recevoir une valeur actuelle du signal physiologique ;
démarrer un compteur a ajouter une valeur unitaire au compteur a ;
comparer la valeur actuelle du signal physiologique à une valeur mémorisée ;
si la valeur actuelle du signal physiologique est inférieure à la valeur inscrite, remplacer la valeur mémorisée par la valeur du signal physiologique, ajouter une valeur de décompte du compteur a à un accumulateur b, et effacer le compteur a à zéro, et recevoir une valeur suivante du signal physiologique ;
si la valeur actuelle du signal physiologique est plus grande que la valeur mémorisée, recevoir une valeur suivante du signal physiologique ;

déterminer que la valeur correspondante du signal physiologique est une valeur minimum quand la valeur de décompte du compteur a atteint une valeur correspondant à un second temps préétabli ;
délivrer une valeur accumulée dans l'accumulateur b, effacer le compteur a, la mémoire, et l'accumulateur b ;
redémarrer la procédure de reconnaissance de valeur minimum et déterminer une valeur minimum suivante ; et
obtenir un second cycle du signal physiologique en ajoutant un temps requis pour atteindre la valeur accumulée dans l'accumulateur b et le second temps préétabli ;

le procédé comprenant en outre les étapes suivantes consistant à :

juger quant à savoir si le premier cycle et le second cycle respectivement obtenus dans une durée temporelle sont proches l'un de l'autre ; et
si le premier cycle et le second cycle sont proches l'un de l'autre, déterminer qu'une moyenne des deux cycles est le cycle du signal physiologique,

dans lequel la condition que le premier cycle et le second cycle sont proches l'un de l'autre signifie que le premier cycle et le second cycle présentent une différence temporelle de 0,004 seconde si le signal physiologique et un signal de respiration ou présentent une différence temporelle de 0,017 seconde si le signal physiologique est un signal de battement cardiaque.

2. Procédé selon la revendication 1, dans lequel le premier temps préétabli et le second temps préétabli sont plus longs qu'un demi-cycle d'une limite supérieure d'une plage de reconnaissance du cycle du signal physiologique.

3. Système pour obtenir un cycle d'un signal physiologique en exécutant le procédé selon la revendication 1 ou 2, comprenant :

une unité de jugement de valeur extrême, configurée pour exécuter la procédure de reconnaissance de valeur maximum et la procédure de reconnaissance de valeur minimum ;
une unité de calcul de cycle, configurée pour obtenir le premier cycle du signal physiologique et le second cycle du signal physiologique,
une unité de jugement de proximité de cycle, configurée pour juger quant à savoir si le premier cycle et le second cycle respectivement obtenus dans une durée temporelle sont proches l'un de l'autre ; et
une unité de détermination de cycle, configurée pour déterminer qu'une moyenne des deux cycles est le cycle du signal physiologique si le premier cycle et le second cycle sont proches l'un de l'autre.

FIG. 1

FIG. 2

```
                        ⬡ Start ⬡
                            │
                            ▼
┌──────────────────────────────────────────────────────┐
│  Receiving a physiological signal value and a         │ ─── S10
│  register value, comparing the physiological signal   │
│  value with the register value, and reserving one     │
│  of the physiological signal value and the register   │
│  value; determining the physiological signal value    │
│  with a duration time thereof reaching a given        │
│  set time to be an extreme value; and restarting      │
│  the procedure and determining a next extreme value   │
└──────────────────────────────────────────────────────┘
                            │
                            ▼
┌──────────────────────────────────────────────────────┐
│  Obtaining a cycle of the physiological signal by     │ ─── S11
│  calculating a time difference between the extreme     │
│  value and the next extreme value.                    │
└──────────────────────────────────────────────────────┘
                            │
                            ▼
                     (  The end  )
```

FIG. 3

Start

Receiving a physiological signal value and a register value, comparing the physiological signal value with the register value, and reserving one of the physiological signal value and the register value; determining the physiological signal value with a duration time thereof reaching a given set time to be an extreme value; and restarting the procedure and determining a next extreme value    — S10

Obtaining a cycle of the physiological signal by calculating a time difference between the extreme value and the next extreme value.    — S11

Judging whether two cycles respectively obtained in the maximum value way and in the minimum value way in a time duration are close to each other    — S12

If the two cycles are close to each other, determining an average of the two cycles to be the cycle of the physiological signal    — S13

The end

FIG. 4

20

Extreme value
judging unit 21

Cycle calculating
unit 22

FIG. 5

20

Extreme value
judging unit 21

Cycle calculating
unit 22

Close cycle
judging unit 23

Cycle setting unit 24

FIG. 6

**EP 2 752 154 B1**

## REFERENCES CITED IN THE DESCRIPTION

### Patent documents cited in the description

- US 2004186388 A1
- US 2009204011 A1
- JP 2007181628 A